# EUROPEAN PATENT SPECIFICATION

(11) **EP 3 081 952 B1**
(45) Date of publication and mention of the grant of the patent: **26.12.2018**
(21) Application number: 15164035.6
(22) Date of filing: 17.04.2015
(51) Int. Cl.: G01R 33/28, G01R 33/46, G01R 33/56

(54) **TRANSPORTABLE LONG-LIVED HYPERPOLARIZED SAMPLES**
TRANSPORTABLE LANGLEBIGE HYPERPOLARISIERTE PROBEN
ÉCHANTILLONS TRANSPORTABLES HYPERPOLARISÉS À LONGUE VIE

(43) Date of publication of application: 19.10.2016
(73) Proprietor: ECOLE POLYTECHNIQUE FEDERALE DE LAUSANNE (EPFL), 1015 Lausanne (CH)
(72) Inventor: Jannin, Sami Antoine Adrien, 1026 Echandens (CH); Bodenhausen, Geoffrey, 75014 Paris (FR); Emsley, Lyndon, 1004 Lausanne (CH)
(74) Representative: Bremi, Tobias Hans

(56) References cited:
- D. GAJAN ET AL: "Hybrid polarizing solids for pure hyperpolarized liquids through dissolution dynamic nuclear polarization", PROCEEDINGS OF THE NATIONAL ACADEMY OF SCIENCES, vol. 111, no. 41, 29 September 2014 (2014-09-29), pages 14693-14697, XP055204704, ISSN: 0027-8424, DOI: 10.1073/pnas.1407730111
- LAFON O ET AL: "Mesoporous silica nanoparticles loaded with surfactant: Low temperature magic angle spinning <13>C and <29>Si NMR enhanced by dynamic nuclear polarization", JOURNAL OF PHYSICAL CHEMISTRY C 20130131 AMERICAN CHEMICAL SOCIETY USA, vol. 117, no. 4, 31 January 2013 (2013-01-31), pages 1375-1382, XP002742727,
- ROSSINI A J ET AL: "Dynamic nuclear polarization NMR spectroscopy of microcrystalline solids", JOURNAL OF THE AMERICAN CHEMICAL SOCIETY 20121010 AMERICAN CHEMICAL SOCIETY USA, vol. 134, no. 40, 10 October 2012 (2012-10-10), pages 16899-16908, XP002742728, DOI: 10.1021/JA308135R

## Description

### TECHNICAL FIELD

The present invention relates to a method for the production of long-lived hyperpolarized samples, as well as to uses of such a method for measurement purposes.

### PRIOR ART

Dissolution dynamic nuclear polarization (D-DNP) allows one to increase the NMR polarization and hence the signals of molecules labeled with isotopes that have a nuclear magnetic moment such as ¹³C, ¹⁵N, ²⁹Si, ³¹P, ⁶Li and ⁸⁹Y, etc., by several orders of magnitude by performing low temperature DNP followed by rapid melting, dissolution and heating to room temperature. Molecules can be "hyperpolarized" by boosting the polarization of their nuclei by DNP. The production of hyperpolarized molecules of interest such as metabolites in bulk opens the way to a broad diversity of novel applications, such as the detection of intermediates in chemical reactions, the observation of protein folding in real time, or the monitoring of cancer in humans. In order to deliver hyperpolarized samples to the system under investigation (be it an NMR tube or a human), the melting or dissolution step is normally carried out in the magnetic field of the DNP polarizer, and the sample must be rapidly injected, within about 1 minute, depending on T₁(¹³C). If these two conditions are fulfilled, most of the hyperpolarized magnetization can be preserved.

The need to melt or dissolve the sample within the polarizer is a major limitation. Because polarization in solution only lasts for a few minutes, it introduces the need for a polarizer at the point of use. Solid-state relaxation properties are very different from those in solution, so removal of the sample in the solid state might in principle allow the polarization to be preserved for long times. However, any attempts to take the hyperpolarized solid sample out of the polarizer and melt it in a remote place also appear inevitably doomed to fail. The reason for this limitation is that, as shown in the experimental section further below, ¹³C nuclear spin-lattice relaxation times in DNP samples become prohibitively short in low magnetic fields because relaxation is exacerbated by the presence of the paramagnetic polarizing agents (PAs) that are indispensable for DNP. The problem therefore appeared unresolvable so far.

In D-DNP experiments, the ¹³C-labeled metabolites are usually polarized at very low temperatures (1.2 < T < 4.2 K) and moderate fields (usually B₀ ranges from 3.35 to 6.7 T) either directly, or indirectly by ¹H→¹³C cross polarization. One feature of DNP is that it is usually done with a statistical distribution of PAs in a frozen glassy solution of the substrate of interest, which is normally achieved by shock-freezing aqueous mixtures of PAs and ¹³C-labeled metabolites, possibly mixed with a glass-forming agent such as glycerol. For efficient DNP the frozen samples must be glassy and transparent. Samples turn opaque when crystallization occurs, leading to a partial separation of the metabolites and PAs, and DNP becomes very inefficient. Alternatively, the PAs can be covalently attached to the surface of mesostructured materials that are impregnated with metabolite solutions, or generated in-situ by UV irradiation. However, in all of these strategies paramagnetic relaxation in low fields precludes carrying the hyperpolarized samples over large distances for remote D-DNP.

Gajan et al in PNAS, Vol 11, No 41, pages 14693-14697 report on hyperpolarization of substrates for magnetic resonance spectroscopy and imaging by dissolution dynamic nuclear polarization using HYPSO-1.0 impregnated with a carbon 13 labeled pyruvate solution.

### SUMMARY OF THE INVENTION

Hyperpolarization by dissolution DNP has enabled in vivo metabolic imaging of ¹³C-labeled tracers by MRI. The NMR signals of metabolites can be enhanced by several orders of magnitude. During the limited lifetime of the hyperpolarized magnetization, a variety of observations can be performed, ranging from the detection of tumors in humans such as in prostate cancer, to the monitoring of the response to chemotherapy. Because of the limited lifetime of the hyperpolarized magnetization (typically T₁ < 50 s), the DNP process must be performed on-site, close to the MRI scanner, with sophisticated machinery that must be sterile for applications to humans.

It is thus an object of the present invention to provide a solution to these obstacles and to propose for the first time a method allowing for remote production and long-distance transportation of hyperpolarized metabolites, or more generally hyperpolarized molecules of interest. This is achieved by suitable compartmentalization of the molecules of interest and the polarizing agents so that on the one hand cross-polarization between these two is possible, but on the other hand the effect of the polarizing agents on the relaxation of the molecules of interest can be reduced to a minimum. According to one embodiment this is for example achieved by impregnating microcrystals of molecules of interest, e.g. metabolites, with a glass-forming solvent that contains polarizing agents and cannot dissolve the microcrystals. The proton hyperpolarization produced in the component with polarizing agent (CPA) is then transported to the molecules of interest, e.g. metabolites, contained in the component with the molecule of interest (CMI) by proton spin diffusion, and converted into carbon-13 hyperpolarization by cross-polarization so that it cannot "leak" out of the CMI, which can be transported over long distances during extended times.

To remove the polarized solid from the polarizer, the radicals and the substrate should preferably be physically separated from each other to prevent relaxation causing the decay of the polarization, yet close enough for the radicals to polarize the substrate on the DNP timescale. This seems like a paradox. However, here we show how this can be achieved by formulating the DNP sample with a suitable microarchitecture. The sample can be made of two matrices interleaved on a micrometer scale.

More generally speaking, the present invention relates to a method as summarized in claim 1.

The method for the preparation of a hyperpolarized solution or suspension of molecules of interest in particular comprises at least the following steps:
1) providing a micro-particulate matrix, which can be crystalline or a non-porous aggregate, and which is comprising or consisting of the molecules of interest, or providing a porous aggregate which is impregnated with molecules of interest, and suspending, emulsifying or coating of said a micro-particulate matrix with a solution, emulsion or suspension comprising a DNP-suitable polarizing agent, or surface functionalizing the micro-particulate matrix with a DNP-suitable polarizing agent, at a first temperature at which the micro-particulate matrix is not dissolving; or providing a micro-particulate matrix, which is comprising or consisting of the DNP-suitable polarizing agent and suspending, emulsifying or coating of said a micro-particulate matrix with a solution, emulsion or suspension comprising the molecules of interest, at a first temperature at which the micro-particulate matrix is not dissolving;
2) lowering the temperature to a value of at most 15 K leading to a frozen DNP sample;
3) transferring the electron spin polarization of the polarizing agent in the DNP sample at this low temperature in a magnetic field of at least 2 T to abundant nuclear spins, allowing for spin diffusion to relay polarization to at least one of:
   - abundant nuclear spins in the molecules of interest;
   - in case of the micro-particulate matrix comprising the molecules of interest abundant nuclear spins in the micro-particulate matrix comprising the molecules of interest;
   - in case of a micro-particulate matrix, which is comprising or consisting of the DNP-suitable polarizing agent, abundant nuclear spins in the solvent or emulsion or suspension agent comprising the molecules of interest;
   and performing hetero-nuclear cross-polarization from the abundant nuclear spins of the molecules of interest and/or the abundant nuclear spins in the micro-particulate matrix comprising the molecules of interest or the abundant nuclear spins in the solvent or emulsion or suspension agent comprising the molecules of interest to at least one nuclear spin type different from the abundant nuclear spins in the molecules,
   wherein transferring the electron spin polarization to the abundant nuclear spins includes transferring electron spin polarization to protons of the polarizing agent or of molecules surrounding the polarizing agent and to protons of the molecules of interest or of molecules surrounding the molecules of interest by using microwave irradiation;
4) increasing the temperature and melting, dissolving or suspending the molecules of interest where the nuclear spin type different from the abundant nuclear spins is hyperpolarized, in particular for use in a magnetic resonance imaging or nuclear magnetic resonance experiment.

In respect of the individual steps the following definitions and possible specifications apply:
*1) providing a micro-particulate matrix, which can be crystalline or a non-porous aggregate, and which is comprising or consisting of the molecules of interest, or a porous aggregate which is impregnated with molecules of interest, and suspending, emulsifying or coating of said a micro-particulate matrix with a glass-forming solution, emulsion or suspension comprising a DNP-suitable polarizing agent, or surface functionalizing the micro-particulate matrix with a DNP-suitable polarizing agent, at a first temperature at which the micro-particulate matrix is not dissolving;*
*or providing a micro-particulate matrix, which is comprising or consisting of the DNP-suitable polarizing agent and suspending, emulsifying or coating of said a micro-particulate matrix with a solution, emulsion or suspension comprising the molecules of interest, at a first temperature at which the micro-particulate matrix is not dissolving.*

Preferably this step involves suspending or coating of a micro-particulate matrix, which is crystalline or a non-porous aggregate, and which is comprising or consisting of the molecules of interest, with a glass-forming solution, emulsion or suspension comprising a polarizing agent suitable for DNP at a first temperature at which the micro-particulate matrix is not dissolving.

In an alternative approach it is however also possible to provide for a micro-particulate matrix which is a porous aggregate, and which is deeply impregnated with molecules of interest or with a mixture including the molecule of interest. This micro-particulate matrix can then be either suspended or coated as pointed out above.

Such a micro-particulate matrix in the form of beads can also be surface functionalized with a polarizing agent that may be covalently bound to the surface.

For example possible are experiments, where:
(1) the polarizing agents are covalently attached to micro-beads of a polymer (e.g. polystyrene) or to a micro-porous polymer (e.g. polystyrene), or to a micro-drilled polymer (e.g. polystyrene).
(2) the polymeric micro-particulate matrix is then impregnated with a solution containing the molecule of interest, or even the molecule of interest itself in a liquid form. One most interesting molecule can be pyruvic acid.
(3) after dissolution, the polymer micro-particulate matrix beads can be separated from the solution by filtration.

However in case of a crystalline or non-porous aggregate, functionalizing the surface with the polarizing agent is also possible, simplifying the separation process as pointed out above after hyperpolarization. In case of surface functionalizing, the micro-particulate matrix is simply suspended in a suitable solvent which preferably neither dissolves the aggregates nor the crystalline structures or, in case of a porous aggregate, neither dissolves the porous aggregate nor perturbs the impregnation with the molecule of interest.

In yet another alternative approach it is also possible to provide a micro-particulate matrix (e.g. a polymer matrix, micro-beads), comprising or consisting of the DNP-suitable polarizing agent and suspending, emulsifying or coating of said a micro-particulate matrix with a solution, emulsion or suspension comprising the molecules of interest.

The micro-particulate matrix is to be understood as being either essentially pure micro-crystallites of the molecule(s) of interest or, in the alternative approach, of the polarizing agent or micro-crystallites comprising the molecule of interest (or in the alternative approach the polarizing agent) together with one or several other components which then serve as a crystalline matrix. Also possible are, when talking about non-porous aggregates, micro-particles which are not crystalline but which are for example amorphous, in particular in this case it is further important that these aggregates are not open to being penetrated by the glass-forming solution or suspension comprising a DNP-suitable polarizing agent or in the alternative approach the molecules of interest in order not to allow the polarizing agent to get into close contact to essentially all the molecules of interest in the particles.

It should further be noted that the micro-particulate matrix can be provided in the form of a powder, i.e., in the form of loose micro-particles, but it can also be provided in the form of a packed and coherent structure of these micro-particles which is however sufficiently open for inter-particle penetration by the glass-forming solution in step 1) and for release of the glass-forming solution in step 4) when separating the glass-forming solution/suspension.

What is furthermore to be noted is that at least under the conditions chosen in step 1) the microparticles of the micro-particulate matrix are not dissolving in the glass-forming solution/suspension/emulsion.

The microparticles can be suspended in the glass-forming solution/suspension/emulsion in step 1) but it can however also be sufficient to just coat or impregnate the microparticles in this step, as long as there is sufficient polarizing agent available on the surface for efficient polarization transfer in step 3).

The polarizing agent, which is typically an organic or inorganic molecule with one or several unpaired electrons (inclusive of metal centers with unpaired electrons, possibly with suitable ligands), can be suspended, emulsified preferably dissolved, in a suitable solvent which, as pointed out above, should not, under the conditions chosen in step 1), dissolve the microparticles containing or consisting of the molecules of interest.

In many situations the temperature chosen in step 1) will be room temperature, in any case the temperature in which the solvent comprising the polarizing agent is sufficiently liquid to mix with the microparticles comprising or consisting of the molecules of interest. One can also use lower temperatures or temperatures higher than room temperature. For example the PA can be dissolved in a liquid gas at -10°, or in a liquid polymer at 80°.
*2) lowering the temperature to a value of at most 15 K frozen-DNP-sample;*
   Typically this temperature in step 2) is the same or close to the one used in step 3). The DNP sample is preferably a solid composite/solution/emulsion/suspension with the microparticles of the molecules of interest embedded in the solid composite/solution/emulsion/suspension with the polarizing agent, or in the alternative approach with the microparticles of the polarizing agent embedded in the solid composite/solution/emulsion/suspension with the molecules of interest. In order to make sure that the micro-particulate structures are sufficiently homogeneously distributed in the glassy structure it can be advantageous to stir or move the composite/solution/emulsion/suspension at least in an initial phase of step 2 to avoid agglomeration before freezing.
*3) transferring the electron spin polarization of the polarizing agent in the DNP sample e.g. by microwave irradiation at this low temperature in a magnetic field of at least 2 T to abundant nuclear spins, allowing for spin diffusion to relay polarization to at least one of:*
   *abundant nuclear spins in the molecules of interest;*
   *in case of the micro-particulate matrix comprising the molecules of interest abundant nuclear spins in the micro-particulate matrix comprising the molecules of interest;*
   *in case of the micro-particulate matrix, which is comprising or consisting of the DNP-suitable polarizing agent, abundant nuclear spins in the* composite/solution/emulsion/suspension *comprising the molecules of interest;*
   *and performing hetero-nuclear cross-polarization from the abundant nuclear spins of the molecules of interest and*/*or in the micro-particulate matrix to at least one different nuclear spin type in the molecules of interest;*

The step 3) can be repeated for stepwise polarization enhancement, e.g. 2-20 times, if necessary.

In this 3^{rd} step the high polarization of the electron spins shall be transferred to the different nuclear spins in the molecule of interest. This is normally not efficient directly but is done by 1^{st} transferring the electron polarization to the abundant nuclear spins of the component containing the polarizing agent and/or of the solvent, or the composite/solution/emulsion/suspension agent, and by spin diffusion to the abundant nuclear spins of the component containing the molecule of interest or to the abundant nuclear spins of the molecule of interest itself.

In the alternative approach the electron polarization of the polarizing agent contained in the microparticles is transferred to abundant nuclear spins of either the polarizing agent itself or the matrix in which the polarizing agent is embedded in the microparticles. Further the polarization is transferred to abundant spins of the composite/solution/emulsion/suspension agent with the molecules of interest and/or directly to the molecules of interest.

Then subsequently hetero-nuclear cross-polarization transfer takes place, while polarization transfer can be cross-polarization at high field, or Nuclear Thermal Mixing by shuttling the sample at low field, from the abundant protons to the different nuclear spins, typically carbon-13 or nitrogen-15, or after initial start of DNP with microwave irradiation, the hetero-nuclear cross-polarization is restarted while maintaining the microwave irradiation transferring polarization from the electron spins to the abundant nuclear spins. This process may include continuous irradiation and/or pulsed irradiation for cross-polarization as well as flip back pulses and detection pulses for monitoring purposes.
*4) increasing the temperature and dissolving or suspending the molecules of interest which are hyperpolarized in respect of the different nuclear spins, in particular for use in a magnetic resonance imaging or nuclear magnetic resonance experiment.*

In this step 4) the actual making available of the hyperpolarized molecules of interest (hyperpolarized in terms of e.g. carbon-13 or nitrogen-15 or one of the other nuclei mentioned above) to an experimental setup typically at room temperature is realized. Preferably in order to avoid detrimental relaxation of the hyperpolarization during this step the polarizing agent is separated from the hyperpolarized molecules of interest.

One gist of the present invention thus can be seen in that in one of the embodiments by impregnating microcrystals or essentially non-porous micro-aggregates of molecules of interest (Mol), e.g. metabolites, with a typically glass-forming, e.g. hydrophobic, DNP matrix that contains polarizing agents but cannot dissolve the microcrystals/non-porous micro-aggregates, proton hyperpolarization can be transported to the Mol contained in the microcrystals/non-porous micro-aggregates through spin diffusion, and converted into carbon-13 hyperpolarization by cross-polarization so that it cannot "leak" out of the microcrystals/non-porous micro-aggregates, which consequentially can be transported over long distances during extended times.

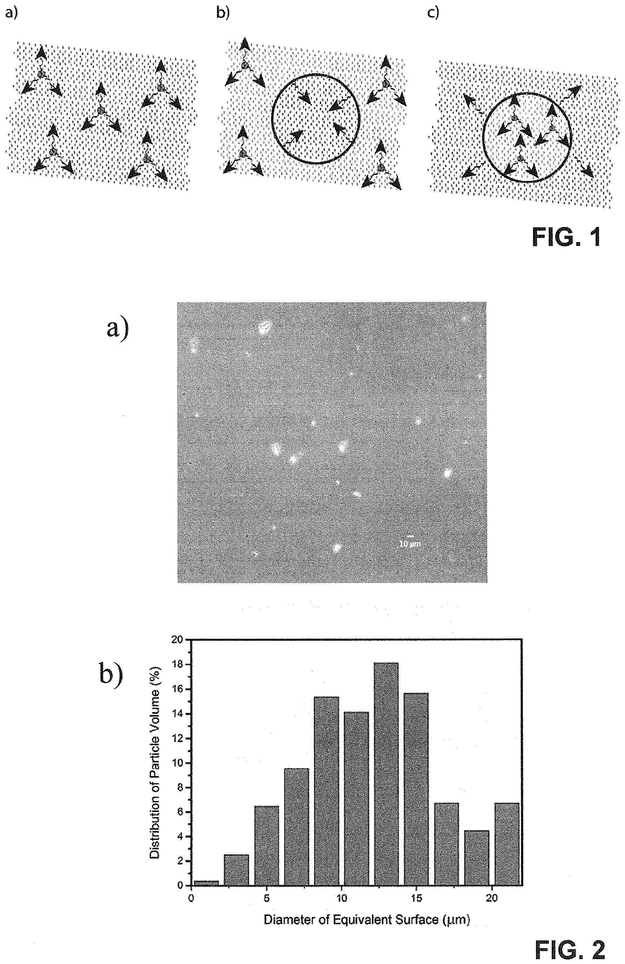

One component that we call the DNP matrix according to one preferred embodiment contains the PAs and abundant spins such as protons, and the other component that we refer to as the micro-crystalline or non-porous micro-aggregate matrix contains the molecules of interest (e.g. metabolites) labeled with ¹³C (and/or any of the other nuclei mentioned above) and containing the same abundant spins. The general idea, illustrated schematically in Figure 1, is that abundant spins such as ¹H are directly and rapidly polarized by DNP in the glassy DNP matrix, and that this polarization is then relayed more slowly to the ¹H of the frozen liquid, micro-crystalline or non-porous micro-aggregate matrix by nuclear spin diffusion. The time needed for spin diffusion to propagate the ¹H polarization from the glassy DNP matrix to the micro-crystalline or non-porous micro-aggregate matrix depends on the dimensions of the microcrystals. We show that 10 µm diameter particles typically translate into a time-scale for spin diffusion of 10 minutes. Once the ¹³C nuclei in the micro-crystalline or non-porous micro-aggregate matrix are surrounded by highly polarized ¹H spins, a ¹H→¹³C cross-polarization sequence is applied to transfer the polarization to the ¹³C spins of the metabolites in the micro-crystalline or non-porous micro-aggregate matrix. At this point, DNP is switched off, and the protons relax towards thermal equilibrium within minutes, while the ¹³C spins, which are no longer in contact with the proton bath or the PAs, remain hyperpolarized for prolonged periods, typically for days. Indeed, at this point the ¹³C hyperpolarization is locked in the micro-crystalline or non-porous micro-aggregate matrix where (i) there are no PAs that could act as relaxing agents, and (ii) the ¹³C hyperpolarization cannot leak back from the micro-crystalline or non-porous micro-aggregate matrix to the glassy DNP matrix since ¹³C spin diffusion is extremely inefficient.

In the alternative approach the roles of the compartments is so to speak reversed, i.e. the microparticles comprise the polarizing agent and the glassy matrix comprises the molecules of interest. One advantage of this alternative approach is that the polarizing agent is attached to the microparticles and upon heating of the corresponding sample separation is easily possible by filtering out the microparticles leaving the solution/suspension of the molecules of interest that are hyperpolarized.

Nano-crystalline suspensions were first polarized by DNP assisted by spin diffusion by van der Wel et al., who used DNP to enhance NMR signals of amyloid fibrils in the vicinity of 100 K in combination with MAS (J. Am. Chem. Soc 2006, 128, 10840-10846). More recently, the idea was generalized to micro-particulate samples, and it was shown that this could lead to substantial polarization in ordinary organic solids or even provide information about domain structures in pharmaceutical formulations. All these studies were performed in magic-angle spinning (MAS) experiments around 100 K.

Capitalizing on the concept of DNP relayed by spin diffusion, we show here inter alia how microcrystals of ¹³C-labelled metabolites (such as [1-¹³C]urea and [1-¹³C]pyruvate) impregnated with a DNP matrix made of glass-forming solutions containing PA's, spin diffusion from the glassy DNP matrix to the microcrystals, followed by cross-polarization from ¹H to ¹³C can lead to a "locked" state of ¹³C polarization (P(¹³C) > 20%). We demonstrate that the hyperpolarization in these microcrystals or non-porous micro-aggregates can have lifetimes greater than 10 hours at 4.2 K. We also show that due to the special nature of these "locked" states, the sample can be removed from the DNP polarizer while preserving much of its polarization.

This opens up possibilities, in opposition to conventional glassy DNP samples, to take the microcrystals out of the polarizer and even transport them to a remote location in a transport device consisting of an ordinary cryogenic Dewar equipped with an insert comprising a permanent magnetic field (here Bₜᵣₐₙₛₚₒᵣₜ > 0.8 T). In the transport device, the typical lifetime of ¹³C hyperpolarization is > 30 minutes. The DNP sample can then be dissolved at a remote location. We show that if the DNP matrix is chosen to be non-miscible with water, the hyperpolarized metabolites (soluble in water) can be easily separated from the PAs and any other contaminants during the dissolution process, yielding a pure hyperpolarized solution without the need for further purification.

For DNP to be efficient, the samples are typically doped with the PAs at concentrations of tens of mM, giving rise to intense electron-electron dipolar couplings, and thus to homogeneous line-widths on the order of tens of MHz. If the magnetic field is lowered to such an extent that the nuclear Larmor frequency of the ¹³C spins falls below the homogeneous dipolar width, the nuclear spin system is said to be in 'thermal contact' with the electron dipolar spin bath, and relaxes with its dipolar relaxation time constant T_{1D}, which usually does not exceed a few tens of ms. The more intimate the contact of the PAs with the ¹³C labelled metabolites, the shorter the relaxation times.

According to a first preferred embodiment of the proposed method, the particles of the micro-particulate matrix have an average particle size in the range of 3-30 µm, preferably in the range of 5-20 µm, particularly preferably in the range of 8-15 µm. If the particle sizes are chosen in this range there is an optimum balance between the contact between the micro-particulate matrix and the polarizing agents during step 3) but also a sufficient separation between the molecules of interest and the polarizing agents to avoid detrimental relaxation of the polarization established in the molecules of interest during step 3) by the unpaired electrons of the polarizing agents.

According to another preferred embodiment, the temperature in steps 2) and/or 3) is at most 10 K, preferably at most 5 K, particularly preferably at most 2 K. Typically the temperatures are in the range of 1-2 K.

The static magnetic field in step 3) is preferably chosen at least 0.1 T, preferably at least 3 T or 4 T, particularly preferably at least 6 T.

Transferring the electron spin polarization to the abundant nuclear spins includes transferring electron spin polarization to protons of the the polarizing agent or of molecules surrounding the polarizing agent and to protons of the molecules of interest or of molecules surrounding the molecules of interest by using microwave irradiation, which is relayed to the protons of the component containing the molecules of interest and/or of the molecule itself or other molecules by nuclear spin diffusion.

The hetero-nuclear cross-polarization in step 3) according to another preferred embodiment includes pulsed and/or continuous radiofrequency irradiation essentially at the Larmor frequencies of both the abundant nuclear spins and the different nuclear spin type.

The nuclear spin type different from the abundant nuclear spin is preferably selected from the group consisting of ¹³C, ¹⁵N, ²⁹Si, ³¹P, ⁶Li and ⁸⁹Y, and the molecules of interest can be correspondingly labeled.

Within step 4), preferably before solution/suspension, the molecules of interest are separated from the polarizing agent. This can be done in that first the temperature is raised in the sample until the glass-forming solution or suspension comprising the polarizing agent is in the liquid state but the micro-particulate matrix is still in the solid state, separating the micro-particulate matrix from the glass-forming solution or suspension or at least from the polarizing agent contained therein, preferably using filtering, centrifugation, sedimentation, chromatography or a combination thereof, and dissolving the isolated micro-particulate matrix and/or the molecules of interest.

In the alternative approach where the polarizing agent is contained in the microparticles, separation can be carried out by heating the samples until the solution/suspension of the molecules of interest is sufficiently liquid to filter out the microparticles containing the polarizing agent, making a solution/suspension of hyperpolarized molecules of interest directly available.

In this manner, essentially pure hyperpolarized molecules of interest can be obtained, well separated from the relaxation-active polarizing agents allowing for longer storage and/or more extended experiments.

According to yet another preferred embodiment of the proposed method, after step 3) and before step 4) the sample is transported to a remote location, said transport involving taking the sample temporarily out of the magnetic field of step 3).

This is one of the key advantages of the proposed method, namely due to the shielding of the hyperpolarized molecules in the microparticles from the relaxing effect of the polarizing agents in the component containing the PA, the hyperpolarization is much more stable over time, since relaxation is much slower. Due to this it is even possible to transport the sample to a remote location and/or take it briefly (typically for not more than 10 seconds) out of the magnetic field.

Another preferred embodiment of the proposed method is characterized in that the solution or suspension comprising a DNP-suitable polarizing agent comprises the DNP-suitable polarizing agent at a concentration of at least 5 mM, preferably at least 10 mM, more preferably in the range of 10-50 mM.

The DNP-suitable polarizing agent can be selected from nitroxide derivatives, preferably from the group consisting of TEMPO, TEMPOL, TEMPO-benzoate, or a mixture thereof, but also other radicals such as BDPA, DPPH, galvinoxyl and other polarizing agents containing rare earth elements.

The solvent and/or suspending liquid of the solution or suspension comprising a DNP-suitable polarizing agent can be selected from a group comprising or consisting of tetrahydrofurane, dioxane, hexane, heptane, octane, nonane, benzene, toluene, DMSO, water, glycerol or a mixture thereof.

Furthermore the molecule of interest can be selected from the group comprising or consisting of urea, pyruvate, fumarate, malate, glucose, acetate, alanine, or a mixture thereof.

In step 4) the molecule of interest can for example be dissolved in water or in a physiological liquid. Also possible is however a different liquid.

Further embodiments of the invention are laid down in the dependent claims.

### BRIEF DESCRIPTION OF THE DRAWINGS

Preferred embodiments of the invention are described in the following with reference to the drawings, which are for the purpose of illustrating the present preferred embodiments of the invention and not for the purpose of limiting the same. In the drawings,
- Fig. 1: shows an illustration of the DNP mechanism in (a) a conventional glassy DNP solution containing both metabolites (small dots) and polarizing agents (filled circles); (b) micro-crystals containing only metabolites but no polarizing agents, coated or covered by a DNP solution containing only polarizing agents but no metabolites; (c) polymer containing the polarizing agents, covered by a solution containing the metabolites;
- Fig. 2: shows in (a) a microscope image of micro-crystals of [1-¹³C]urea after manual grinding, and in (b) a distribution of the particle size of these micro-crystals;
- Fig. 3: shows the DNP build-up of the proton NMR signal intensity that is proportional to the proton polarization P(¹H) in (a) a glass-forming DNP solution and (b) in microcrystals that are impregnated in a glass-forming DNP solution, where the fast build-up is associated with the glass-forming DNP solution, and the slow component is associated with the microcrystals;
- Fig. 4: shows the DNP build-up of ¹³C polarization P(¹³C) after cross-polarization (CP) in (empty circles) a glassy DNP solution containing [1-¹³C]urea, and (filled circles) in microcrystals of [1-¹³C]urea that are impregnated in a glass-forming DNP solution;
- Fig. 5: shows ¹³C nuclear spin lattice relaxation at 4.2 K and 6.7 T in (empty circles) a glassy DNP solution containing [1-¹³C]urea, and (filled circles) in microcrystals of [1-¹³C]urea that are impregnated in a glass-forming DNP solution;
- Fig. 6: shows ¹³C nuclear signals in microcrystals of pure pyruvate that are impregnated in a glass-forming DNP solution measured at 4.2 K and 6.7 T before and after brief transport to low fields in the stray field of the NMR magnet;
- Fig. 7: shows a transportation device formed by eight permanent magnets assembled in a Halbach configuration to give a field of 1 T, inserted into a standard liquid helium transport Dewar; the polarized DNP sample can be lowered manually into the Dewar to be transported to a remote location, since T₁(¹³C) > 30 min; and
- Fig. 8: shows a hyperpolarized signal of 20 mg of [1-¹³C]urea after dissolution of the impregnated microcrystals with 5 mL of water in the DNP polarizer, transport through a magnetic tunnel, and injection into a 300 MHz NMR spectrometer; the ¹³C signal was measured every 5 s with 5° pulses; the hyperpolarization decay was fitted with a mono-exponential curve with T₁(¹³C) = 60 s, typical for a radical-free solution.

### DESCRIPTION OF PREFERRED EMBODIMENTS

**Metabolite Component.** [1-¹³C]urea and [1-¹³C]pyruvate were chosen as test substances (molecules of interest) since urea was the first hyperpolarized metabolite produced by Dissolution DNP, and because pyruvate has become the metabolite of reference for in-vivo clinical studies as it provides a reliable and efficient means to detect prostate tumors. However, any metabolite or molecule of interest that can be provided in the form of a powder can be equally suitable for the proposed method.

Here powdered samples of micro-crystalline ¹³C-labelled metabolites were ground by hand down to an average particle diameter of 13 µm with a distribution of ± 5 µm (see figure 2). This protocol was used for small quantities of metabolites, less than 1 g. For larger-scale production, we have obtained good results by e.g. zirconia ball milling. Of course, many other preparation methods are possible, such as precipitation/crystallization from a saturated solution, co-crystallization, spray-drying, or re-crystallization by solvent evaporation, etc. for the production of the microcrystalline or non-porous micro-aggregate forms of the molecules of interest. The molecules of interest can essentially be forming this micro-matrix, or the molecules of interest can be embedded in such a micro-matrix, so the micro-matrix does not necessarily consist of the molecules of interest but can also be a mixture of different molecules of interest or can also be a mixture of a carrier with molecules of interest.

**Polarizing Component.** The microcrystals are impregnated by a glass-forming DNP matrix containing PAs chosen such that the microcrystals are not soluble in the DNP matrix under the chosen conditions. Here we used a DNP matrix consisting of Toluene-d6:THF (8:2) doped with 50 mM TEMPOL-benzoate (4-Hydroxy-2,2,6,6-tetramethylpiperidine-oxyl-benzoate), a hydrophobic derivative of TEMPO. This DNP medium was chosen because: (i) a proton polarization P(¹H) > 50% can be achieved in less than 5 minutes at 1.2 K and 6.7 T (see figure 2a); (ii) none of the metabolites that we have tested (urea, pyruvate, acetate, fumarate, glycine, glucose) are soluble in this mixture; and (iii) the mixture is not miscible with water, and can be extracted on-line after dissolution using a suitable resin filter such as Amberlite® XAD4. Of course, many other combinations of glass-forming solvents and PA's are possible, and may turn out to be effective.

Among other candidates for DNP matrices, the use of butane is possible. The microcrystals of metabolites can be immersed at -5° C in liquid butane doped with TEMPOL-benzoate. During the dissolution process the butane is sublimed and flushed out with helium gas. The PAs precipitate as a powder and can be retained by an in-line 0.22 µm syringe filter

### (Merck Millipore)

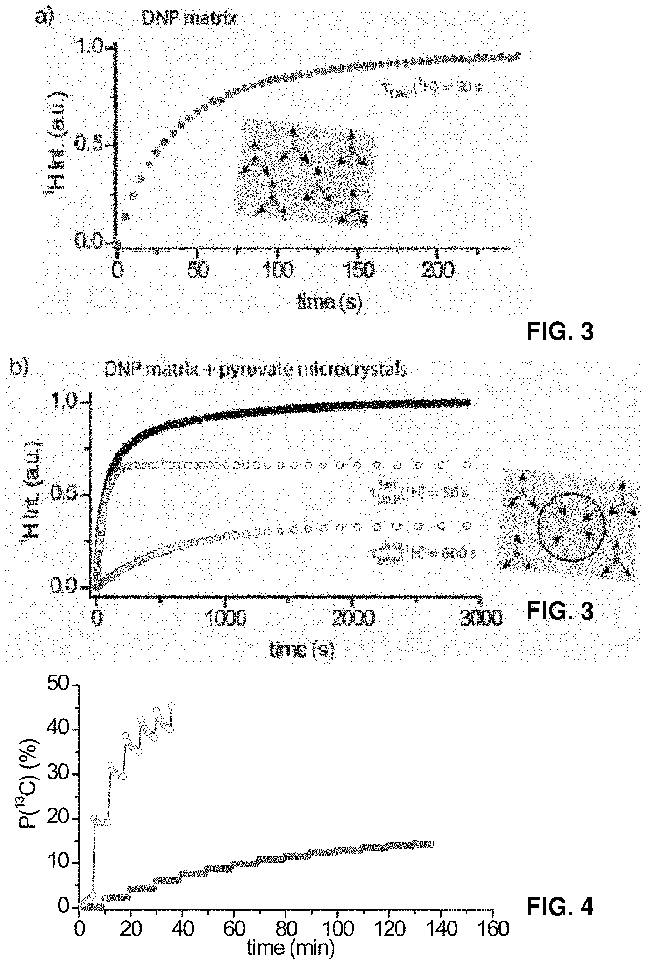

**DNP build-up curves.** Figure 3a shows the ¹H DNP build-up at 1.2 K and 6.7 T for 60 µL of a pure glass-forming DNP matrix consisting of Toluene-d6:THF doped with 50 mM of TEMPOL-benzoate, without any microcrystals of the molecules of interest, i.e., without any metabolites. The polarization reaches P(¹H) > 50% after a mono-exponential build-up with a characteristic time constant τ_{DNP}(¹H) = 50 s. Figure 3b shows the ¹H DNP build-up curve under the same conditions by impregnating 15 mg of the micro-crystalline [1-13C]urea described above with the 60 µL of the Toluene-d6:THF:TEMPOL-benzoate P-matrix. The ¹H DNP build-up curve is now bi-exponential with a fast component τ_{DNP}^{fast}(¹H) = 56 s arising from the direct polarization of the glassy DNP matrix as in Figure 3a, and a slow component τ_{DNP}^{slow}(¹H) = 600 s corresponding to the polarization of the microcrystals which builds up through proton spin diffusion from the glassy DNP matrix. This clearly shows that the polarization enhanced by DNP propagates, albeit slowly, from the glassy DNP matrix to the microcrystals. Polarization can be transferred from the protons to the ¹³C spins by cross-polarization at low temperature, e.g. using the methods disclosed by Jannin et al (Chem. Phys. Lett 517 (2011) 234-236).

Figure 4 shows the ¹³C build-up of the magnetization of [1-¹³C]urea obtained by multiple-contact ¹H → ¹³C cross-polarization at 1.2 K, as compared with a similar ¹H → ¹³C cross-polarization build-up measured on the same amount of [1-¹³C]urea, but dissolved in the conventional glassy matrix H₂O:D₂O:glycerol-d₈ (2:3:5) doped with 40 mM TEMPOL, known as "DNP juice". Although these two ¹H → ¹³C cross-polarization build-up curves might look similar at first glance, it is worth stressing two major differences, (i) the time between two consecutive cross-polarization contacts should be extended (from Δt^{CP} = 5 minutes to 20 minutes) to ensure that ¹H spin diffusion spreads polarization uniformly inside the crystals between cross-polarization steps, and (ii) ¹³C relaxation between cross-polarization contacts, which usually limits the ¹³C build-up, is strongly attenuated in the micro-crystals because of the absence of PAs. These two new features imply that ¹³C polarization is slower to build up but can achieve higher final polarization levels, theoretically up to P(¹³C) = P(¹H).

**Extended relaxation times.** Although our micro-crystalline suspension in the glassy DNP matrix is different from the usual glassy solutions, ¹³C CP-DNP can be performed essentially in the same way. One primary novelty associated with this new sample formulation resides in the dramatically different ¹³C spin-lattice relaxation properties. The ¹³C spins of metabolites dispersed in glassy matrices are inevitably in dipolar contact with the PAs that lead to paramagnetic relaxation. In the microcrystalline formulation on the other hand, the ¹³C spins of the metabolites are physically separated from the PAs on a length scale much larger than that of the electron-nuclear dipolar interaction.

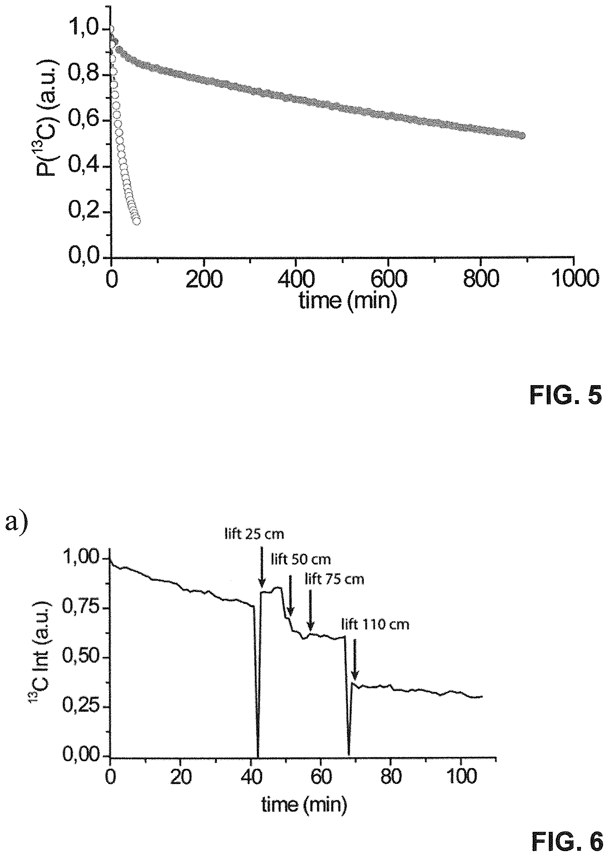

Figure 5 shows how the ¹³C nuclear spin lattice relaxation at 4.2 K and 6.7 T in [1-¹³C]urea is dramatically extended by switching from the glassy solvent where T₁(¹³C) = 20 minutes to the microcrystalline approach where it becomes 37 hours.

**Transport.** This extension of the nuclear spin lattice relaxation time T₁(¹³C) is remarkable. The comparison is likely to be even more favorable in solutions at low fields where solid-state paramagnetic relaxation is exacerbated. The extension of T₁(¹³C) enables transport to remote locations using a simple transport device that maintains a low magnetic field to preserve the polarization.

Figure 6 shows how the ¹³C polarization can even be transported to a low magnetic field (B₀ < 10 mT) at room temperature for brief (t < 10 s) periods of time, whereas the polarization vanishes entirely in glassy samples that contain PAs. Obviously, this feature enables facile manual handling and transportation of a polarized sample from the polarizer (in our case at 6.7 T) to a transport device.

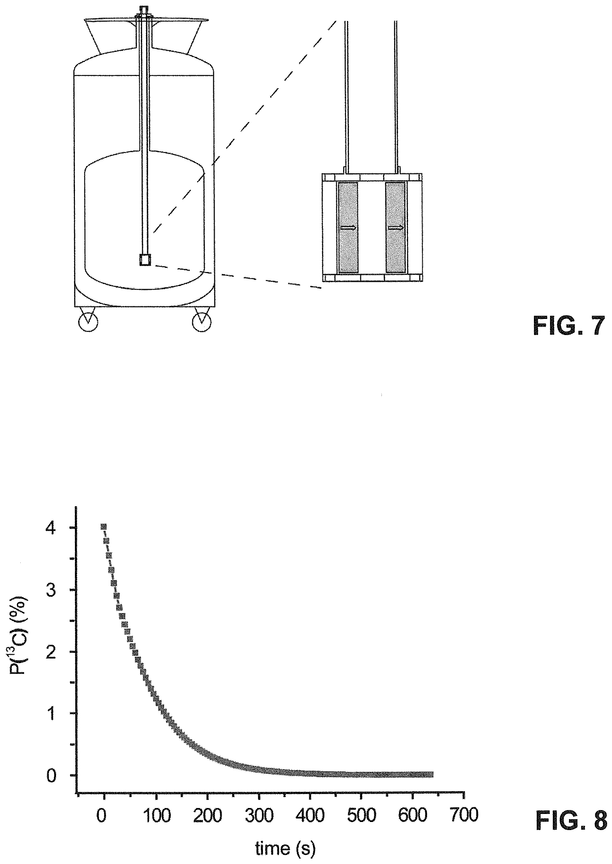

Figure 7 shows a drawing of a transport device equipped with a sample insert with permanent magnets that give a field of 0.8 T, and its accompanying dissolution insert.

**Dissolution.** The thermodynamics of dissolution is always advantageous since (i) the heat capacity C_{P} and latent heat of fusion L_{V} of organic solvents are significantly smaller than those of water, and (ii) the enthalpy of solution ΔHₛₒₗₙ of most organic crystals is negative, so that dissolution is exothermic. Dissolution can therefore be performed by conventional means, without modification of existing devices, and possibly with a smaller dilution factor. The resulting hyperpolarized solutions were pushed through a magnetic tunnel and injected into a 300 MHz liquid-state NMR spectrometer. The dissolution was performed either directly in the DNP polarizer or in the transport device with permanent magnets that give a field of 0.8 T in a helium Dewar.

Figure 8 shows the hyperpolarization decay measured in [1-¹³C]urea, featuring a polarization P(¹³C) = 4 %, corresponding to an enhancement factor ε_{DNP} = 8000 with respect to thermal equilibrium. Interestingly, the ¹³C spin-lattice relaxation time T₁(¹³C) = 60 s is typical for a radical-free solution. This is evidence that the TEMPO-benzoate remains mostly in the organic phase, while the urea dissolves as expected in the aqueous phase.

Conclusions: We have demonstrated a novel approach to Dissolution DNP (D-DNP) dubbed Remote-DNP (R-DNP) that can produce transportable hyperpolarized micro-crystalline samples of pure metabolites free of paramagnetic polarizing agents. The lifetime of the hyperpolarization in the micro-crystalline samples studied here lies in the range between 30 minutes and 30 hours, thus enabling transportation of hyperpolarized micro-crystalline samples to remote locations in suitable transport devices, here an assembly of permanent magnets immersed in liquid helium.

The R-DNP method comprises:
(i) the embedding of hydrophilic microcrystals of pure metabolites in hydrophobic glass-forming DNP solutions that do not dissolve the micro-crystals,
(ii) freezing to ca. 1.2 K,
(iii) proton DNP followed by spin diffusion of proton polarization from the frozen glassy DNP solutions to the microcrystals of metabolites
(iv) ¹H → ¹³C cross-polarization (CP) in the microcrystals,
(v) optional transportation to a remote location in suitable transport device, and finally
(vi) dissolution to the liquid state and injection in an MRI or NMR system.

### Methods:

Sample preparation: the metabolites as well as the solvents and polarizing agents were purchased from Sigma Aldrich. The metabolites powders were ground by hand in a ceramic mortar during ca. 5 minutes.

Microscope and Particle distribution analysis: Pictures of the ground metabolites were taken with a suitable microscope, and the particle size distribution analysis was performed with the freeware ImageJ.

Low-temperature DNP and ¹H → ¹³C cross polarization: Samples were polarized at liquid helium temperatures (1.2 to 4.2 K) and in a magnetic field of 6.7 T in a home-built DNP polarizer. The microwave frequency used was 188.3 GHz with a power of about 80 mW. Cross-polarization sequences were applied with dedicated NMR coils coupled to a Bruker NMR spectrometer. The NMR coils were tuned and matched to 285.2 MHz for protons and 71.7 MHz for carbon-13. The CP pulse sequence consisted in two 180° WURST pulses swept through 100 kHz in 1 milliseconds, and with a *B*₁ strength of about 20 kHz, simultaneously applied to the proton and carbon-13 channels. These CP sequences were repeated several times, at intervals typically varying from 5 to 20 minutes.

Dissolution experiment: the polarized samples were dissolved with 5 mL of preheated deuterated water (at about 450 K at a pressure of 1 MPa). The water was then sprayed onto the sample and the resulting solution was pushed by helium gas at a pressure 0.6 MPa through a 1.5 mm tube through a magnetic tunnel towards the NMR spectrometer, and injected into the NMR tube.

Liquid-state NMR: The ¹³C signals of the hyperpolarized sample were measured by applying 5° nutation angle pulses every 5 seconds.

**LIST OF REFERENCE SIGNS AND ABBREVIATIONS**

| | | | |
|---|---|---|---|
| NMR | Nuclear magnetic resonance | | 13, enriched or in natural |
| MRI | Magnetic resonance imaging | | abundance) and abundant |
| DNP | Dynamic Nuclear Polarization | | spins (e.g., protons that allow spin diffusion to occur.) |
| D-DNP | Dissolution DNP | DNP matrix | ("DNP juice") glassy matrix |
| R-DNP | Remote DNP | | that contains the PAs and |
| CP | Cross Polarization | | abundant spins such as |
| CPA | Component with polarizing agent | MOI | protons for spin diffusion) Molecule of Interest |
| CMI | Component with Molecule of Interest | HYPSO | (metabolite, etc) Hybrid Polarizing Solid |
| PA | polarizing agent (e.g. stable mono-or biradical) | FDA | Federal Drug Administration |
| | | TEMPOL | 4-hydroxy-2,2,6,6-tetramethylpiperidin-1-oxyl |
| Micro-crystalline matrix | | | |
| | ("microcrystals") that contains the MOI with nuclei to be polarized (e.g. carbon- | TEMPO | (2,2,6,6-Tetramethylpiperidin-1-yl)oxyl |

## Claims

1. Method for the preparation of a hyperpolarized solution or suspension of molecules of interest comprising the following steps:
1) providing a micro-particulate matrix, which is crystalline or a non-porous aggregate, and which is comprising or consisting of the molecules of interest, or providing a porous aggregate which is impregnated with molecules of interest, and suspending, emulsifying or coating of said micro-particulate matrix with a solution, emulsion or suspension comprising a DNP-suitable polarizing agent, or surface functionalizing the micro-particulate matrix with a DNP-suitable polarizing agent, at a first temperature at which the micro-particulate matrix is not dissolving;
or providing a micro-particulate matrix, which is comprising or consisting of the DNP-suitable polarizing agent and suspending, emulsifying or coating of said a micro-particulate matrix with a solution, emulsion or suspension comprising the molecules of interest, at a first temperature at which the micro-particulate matrix is not dissolving;
2) lowering the temperature to a value of at most 15 K leading to a frozen DNP sample;
3) transferring the electron spin polarization of the polarizing agent in the DNP sample at this low temperature in a magnetic field of at least 2 T to abundant nuclear spins, allowing for spin diffusion to relay polarization to at least one of:
abundant nuclear spins in the molecules of interest;
in case of the micro-particulate matrix comprising the molecules of interest to abundant nuclear spins in the micro-particulate matrix comprising the molecules of interest;
in case of the micro-particulate matrix, which is comprising or consisting of the DNP-suitable polarizing agent, to abundant nuclear spins of the solvent/emulsion or suspension agent comprising the molecules of interest;
and performing hetero-nuclear cross-polarization from the abundant nuclear spins of the molecules of interest and/or in the micro-particulate matrix to at least one different nuclear spin type in the molecules of interest,
wherein transferring the electron spin polarization to the abundant nuclear spins includes transferring electron spin polarization to protons of the polarizing agent or of molecules surrounding the polarizing agent and to protons of the molecules of interest or of molecules surrounding the molecules of interest by using microwave irradiation;
4) increasing the temperature and melting, dissolving or suspending the molecules of interest which are hyperpolarized with respect of the different nuclear spins, in particular for use in a magnetic resonance imaging or nuclear magnetic resonance experiment.

2. Method according to claim 1, wherein step 1) involves suspending or coating of a micro-particulate matrix, which is crystalline or a non-porous aggregate, and which is comprising or consisting of the molecules of interest, with a glass-forming solution or suspension comprising a DNP-suitable polarizing agent at a first temperature at which the micro-particulate matrix is not dissolving.

3. Method according to claim 1, wherein step 1) involves providing a micro-particulate matrix, which is comprising or consisting of the DNP-suitable polarizing agent and suspending, emulsifying or coating of said a micro-particulate matrix with a solution, emulsion or suspension comprising the molecules of interest, at a first temperature at which the micro-particulate matrix is not dissolving, and wherein step 4) involves increasing the temperature until the solution, emulsion or suspension comprising the molecules of interest is liquid and filtering out the micro-particulate matrix, which is comprising or consisting of the DNP-suitable polarizing agent.

4. Method according to any of the preceding claims, wherein the particles of the micro-particulate matrix have an average particle size in the range of 3-30 µm, preferably in the range of 5-20 µm, particularly preferably in the range of 8-15 µm.

5. Method according to any of the preceding claims, wherein the temperature in steps 2) and/or 3) is at most 10 K, preferably at most 5 K, particularly preferably at most 2 K.

6. Method according to any of the preceding claims, wherein the static magnetic field in step 3) is at least 2 T, preferably at least 3 T or 4 T, particularly preferably at least 6 T.

7. Method according to any of the preceding claims, wherein the hetero-nuclear cross-polarization in step 3) includes pulsed and/or continuous radiofrequency irradiation essentially at the Larmor frequencies of both the abundant nuclear spins and the different nuclear spin type, wherein preferably the different nuclear spin type is selected from the group consisting of ¹³C, ¹⁵N, ²⁹Si, ³¹P, ⁶Li and ⁸⁹Y, and wherein further preferably the molecules of interest are enriched in this different nuclear spin type.

8. Method according to any of the preceding claims, wherein within step 4) the molecules of interest are separated from the polarizing agent, preferably in that first the temperature is raised until the glass-forming solution or suspension comprising the polarizing agent or the molecule of interest is in the liquid state but the micro-particulate matrix is still in the solid-state, to allow separation of the micro-particulate matrix from the glass-forming solution or suspension or at least from the polarizing agent contained therein, preferably using filtering, centrifugation, sedimentation, chromatography or a combination thereof, and dissolving the isolated micro-particulate matrix and/or molecules of interest.

9. Method according to any of the preceding claims, wherein after step 3) and before step 4) the sample is transported to a remote location, said transport involving taking the sample temporarily out of the magnetic field of step 3).

10. Method according to any of the preceding claims, wherein the solution or suspension comprising a DNP-suitable polarizing agent comprises the DNP-suitable polarizing agent at a concentration of at least 5 mM, preferably at least 10 mM, more preferably in the range of 10-50 mM,
and/or wherein the DNP-suitable polarizing agent is selected from nitroxide derivatives, preferably from the group consisting of TEMPO, TEMPOL, TEMPO-benzoate, but also other radicals such as BDPA, DPPH, galvinoxyl, or a mixture thereof.

11. Method according to any of the preceding claims, wherein a solvent and/or suspending liquid is selected from the group consisting of tetrahydrofurane, dioxane, butane, hexane, heptane, octane, nonane, benzene, toluene, DMSO, water, glycerol or a mixture thereof.

12. Method according to any of the preceding claims, wherein the molecule of interest is selected from the group consisting of urea, pyruvate, fumarate, malate, glucose, acetate, alanine or a mixture thereof.

13. Method according to any of the preceding claims, wherein in step 4) the molecule of interest is dissolved in water or in a physiological liquid.

## Patentansprüche

1. Verfahren zur Herstellung einer hyperpolarisierten Lösung oder Suspension von interessierenden Molekülen, umfassend die folgenden Schritte:
1) Bereitstellen einer mikropartikulären Matrix, die kristallin oder ein nicht-poröses Aggregat ist und die die interessierenden Moleküle enthält oder aus ihnen besteht, oder Bereitstellen eines porösen Aggregats, das mit interessierenden Molekülen imprägniert ist, und Suspendieren, Emulgieren oder Beschichten der mikropartikulären Matrix mit einer Lösung, Emulsion oder Suspension, die ein DNP-taugliches Polarisierungsmittel umfasst, oder Oberflächenfunktionalisieren der mikropartikulären Matrix mit einem DNP-tauglichen Polarisierungsmittel bei einer ersten Temperatur, bei der sich die mikropartikuläre Matrix nicht löst;
oder Bereitstellen einer mikropartikulären Matrix, die das DNP-taugliche Polarisationsmittel enthält oder aus diesem besteht, und Suspendieren, Emulgieren oder Beschichten der mikropartikulären Matrix mit einer Lösung, Emulsion oder Suspension, die die interessierenden Moleküle umfasst, bei einer ersten Temperatur, bei der sich die mikropartikuläre Matrix nicht löst;
2) Absenken der Temperatur auf einen Wert von höchstens 15 K, was zu einer eingefrorenen DNP-Probe führt;
3) Übertragen der Elektronenspinpolarisation des Polarisierungsmittels in der DNP-Probe bei dieser niedrigen Temperatur in einem Magnetfeld von mindestens 2 T auf abundant vorhandene Kernspins, wobei die Spindiffusion zur Weiterleitung der Polarisation an mindestens eine der folgenden ermöglicht wird:
abundant vorhandene Kernspins in den interessierenden Molekülen;
im Falle der mikropartikulären Matrix, die die interessierenden Moleküle enthält, an abundant vorhandene Kernspins in der mikropartikulären Matrix sind, die die interessierenden Moleküle enthalten;
im Falle der mikropartikulären Matrix, die das DNP-taugliche Polarisierungsmittel enthält oder aus diesem besteht, zu abundant vorhandenen Kernspins des Lösungsmittels/Emulsions- oder Suspensionsmittels, das die interessierenden Moleküle umfasst;
und Durchführen einer heteronuklearen Kreuzpolarisation von den abundant vorhandenen Kernspins der interessierenden Moleküle und/oder in der mikropartikulären Matrix zu mindestens einem verschiedenen Kernspintyp in den interessierenden Molekülen,
wobei das Übertragen der Elektronenspin-Polarisation auf die abundant vorhandenen Kernspins das Übertragen der Elektronenspin-Polarisation auf Protonen des Polarisierungsmittels oder von Molekülen, die das Polarisierungsmittel umgeben, und auf Protonen der interessierenden Moleküle oder von Molekülen, die die interessierenden Moleküle umgeben, unter Verwendung von Mikrowellenbestrahlung beinhaltet;
4) Erhöhen der Temperatur und Schmelzen, Lösen oder Suspendieren der interessierenden Moleküle, die in Bezug auf die verschiedenen Kernspins hyperpolarisiert sind, insbesondere zur Verwendung in einer Magnetresonanztomographie oder einem Kernspinresonanzversuch.

2. Verfahren nach Anspruch 1, wobei Schritt 1) das Suspendieren oder Beschichten einer mikropartikulären Matrix, die kristallin oder ein nicht-poröses Aggregat ist und die die interessierenden Moleküle umfasst oder aus ihnen besteht, mit einer glasbildenden Lösung oder Suspension, die ein DNP-taugliches Polarisationsmittel umfasst, bei einer ersten Temperatur, bei der sich die mikropartikuläre Matrix nicht löst, beinhaltet.

3. Verfahren nach Anspruch 1, wobei Schritt 1) das Bereitstellen einer mikropartikulären Matrix, die das DNP-taugliche Polarisierungsmittel enthält oder aus diesem besteht, und das Suspendieren, Emulgieren oder Beschichten der mikropartikulären Matrix mit einer Lösung, Emulsion oder Suspension, die die interessierenden Moleküle enthält, beinhaltet, bei einer ersten Temperatur, bei der sich die mikropartikuläre Matrix nicht löst, und wobei Schritt 4) das Erhöhen der Temperatur beinhaltet, bis die Lösung, Emulsion oder Suspension, die die interessierenden Moleküle enthält, flüssig ist, und das Herausfiltern der mikropartikulären Matrix, die das DNP-taugliche Polarisierungsmittel enthält oder aus diesem besteht.

4. Verfahren nach einem der vorhergehenden Ansprüche, wobei die Partikel der mikropartikulären Matrix eine durchschnittliche Partikelgröße im Bereich von 3-30 µm, vorzugsweise im Bereich von 5-20 µm, besonders bevorzugt im Bereich von 8-15 µm aufweisen.

5. Verfahren nach einem der vorstehenden Ansprüche, wobei die Temperatur in den Schritten 2) und/oder 3) höchstens 10 K, vorzugsweise höchstens 5 K, besonders bevorzugt höchstens 2 K beträgt.

6. Verfahren nach einem der vorstehenden Ansprüche, wobei das statische Magnetfeld in Schritt 3) mindestens 2 T, vorzugsweise mindestens 3 T oder 4 T, besonders bevorzugt mindestens 6 T beträgt.

7. Verfahren nach einem der vorhergehenden Ansprüche, worin die heteronukleare Kreuzpolarisation in Schritt 3) gepulste und/oder kontinuierliche Hochfrequenz-Einstrahlung im Wesentlichen bei den Larmor-Frequenzen sowohl der häufigen Kernspins als auch des unterschiedlichen Kernspintyps beinhaltet, worin vorzugsweise der unterschiedliche Kernspintyp ausgewählt ist aus der Gruppe bestehend aus 13C, 15N, 29Si, 31P, 6Li und 89Y, und worin weiterhin vorzugsweise die interessierenden Moleküle mit diesem unterschiedlichen Kernspintyp angereichert sind.

8. Verfahren nach einem der vorhergehenden Ansprüche, wobei innerhalb von Schritt 4) die interessierenden Moleküle vom Polarisierungsmittel getrennt werden, vorzugsweise dadurch, dass zuerst die Temperatur erhöht wird, bis die glasbildende Lösung oder Suspension, die das Polarisierungsmittel umfasst, oder das interessierende Molekül im flüssigen Zustand ist, aber die mikropartikuläre Matrix noch im festen Zustand ist, um die Trennung der mikropartikulären Matrix von der glasbildenden Lösung oder Suspension oder zumindest von dem darin enthaltenen Polarisationsmittel zu ermöglichen, vorzugsweise unter Verwendung von Filtern, Zentrifugieren, Sedimentation, Chromatographie oder einer Kombination derselben, und das Lösen der isolierten mikropartikulären Matrix und/oder der interessierenden Moleküle.

9. Verfahren nach einem der vorhergehenden Ansprüche, wobei nach Schritt 3) und vor Schritt 4) die Probe an einen entfernten Ort transportiert wird, wobei der Transport das vorübergehende Herausnehmen der Probe aus dem Magnetfeld von Schritt 3) beinhaltet.

10. Verfahren nach einem der vorhergehenden Ansprüche, wobei die Lösung oder Suspension, die ein DNP-taugliches Polarisationsmittel umfasst, das DNP-taugliche Polarisationsmittel in einer Konzentration von mindestens 5 mM, vorzugsweise mindestens 10 mM, bevorzugter im Bereich von 10-50 mM umfasst,
und/oder worin das DNP-taugliche Polarisationsmittel ausgewählt ist aus Nitroxidderivaten, vorzugsweise aus der Gruppe bestehend aus TEMPO, TEMPOL, TEMPO-Benzoat, aber auch aus anderen Resten wie BDPA, DPPH, Galvinoxyl oder einer Mischung davon.

11. Verfahren nach einem der vorstehenden Ansprüche, wobei ein Lösungsmittel und/oder eine suspendierende Flüssigkeit ausgewählt ist aus der Gruppe bestehend aus Tetrahydrofuran, Dioxan, Butan, Hexan, Heptan, Oktan, Nonan, Benzol, Toluol, DMSO, Wasser, Glycerin oder einer Mischung davon.

12. Verfahren nach einem der vorhergehenden Ansprüche, wobei das interessierende Molekül ausgewählt ist aus der Gruppe bestehend aus Harnstoff, Pyruvat, Fumarat, Malat, Glukose, Acetat, Alanin oder einer Mischung davon.

13. Verfahren nach einem der vorhergehenden Ansprüche, wobei in Schritt 4) das interessierende Molekül in Wasser oder in einer physiologischen Flüssigkeit gelöst wird.

## Revendications

1. Procédé pour la préparation d'une solution ou suspension hyperpolarisée de molécules présentant de l'intérêt comprenant les étapes suivantes :
1) l'utilisation d'une matrice microparticulaire, qui est cristalline ou qui est un agrégat non poreux et qui comprend les molécules présentant de l'intérêt ou qui est constituée de celles-ci, ou l'utilisation d'un agrégat poreux qui est imprégné de molécules présentant de l'intérêt, et la mise en suspension, l'émulsification ou l'enduction de ladite matrice microparticulaire avec une solution, émulsion ou suspension comprenant un agent polarisant approprié pour la PDN ou la fonctionnalisation en surface de la matrice microparticulaire avec un agent polarisant approprié pour la PDN, à une première température à laquelle la matrice microparticulaire ne se dissout pas ;
ou l'utilisation d'une matrice microparticulaire, qui comprend l'agent polarisant approprié pour la PDN ou qui est constituée de celui-ci et la mise en suspension, l'émulsification ou l'enduction de ladite matrice microparticulaire avec une solution, émulsion ou suspension comprenant les molécules présentant de l'intérêt, à une première température à laquelle la matrice microparticulaire ne se dissout pas ;
2) l'abaissement de la température à une valeur d'au maximum 15 K ce qui conduit à un échantillon pour PDN congelé ;
3) le transfert de la polarisation de spins électroniques de l'agent de polarisation dans l'échantillon pour PDN à cette basse température dans un champ magnétique d'au moins 2 T à des spins nucléaires abondants, ce qui permet la diffusion de spins pour relayer la polarisation vers au moins l'un de :
les spins nucléaires abondants dans les molécules présentant de l'intérêt ;
dans le cas où la matrice microparticulaire comprend les molécules présentant de l'intérêt vers les spins nucléaires abondants dans la matrice microparticulaire comprenant les molécules présentant de l'intérêt ;
dans le cas de la matrice microparticulaire, qui comprend l'agent polarisant approprié pour la PDN ou qui est constituée de celui-ci, vers des spins nucléaires abondants du solvant/agent d'émulsion ou de suspension comprenant les molécules présentant de l'intérêt ;
et la mise en oeuvre d'une polarisation croisée hétéronucléaire des spins nucléaires abondants des molécules présentant de l'intérêt et/ou dans la matrice microparticulaire vers au moins un type de spin nucléaire différent dans les molécules présentant de l'intérêt,
le transfert de la polarisation de spins électroniques vers les spins nucléaires abondants comprenant le transfert de la polarisation de spins électroniques vers des protons de l'agent de polarisation ou de molécules entourant l'agent de polarisation et vers des protons des molécules présentant de l'intérêt ou de molécules entourant les molécules présentant de l'intérêt par l'utilisation de l'exposition à des micro-ondes ;
4) l'augmentation de la température et la fusion, la dissolution ou la mise en suspension des molécules présentant de l'intérêt qui sont hyperpolarisées en ce qui concerne les spins nucléaires différents,
en particulier destiné à être utilisé dans une imagerie par résonance magnétique ou une expérience de résonnance magnétique nucléaire.

2. Procédé selon la revendication 1, dans lequel l'étape 1) comporte la mise en suspension ou l'enduction d'une matrice microparticulaire, qui est cristalline ou qui est un agrégat non poreux et qui comprend les molécules présentant de l'intérêt ou qui est constituée de celles-ci, avec une solution ou suspension formant du verre comprenant un agent polarisant approprié pour la PDN à une première température à laquelle la matrice microparticulaire ne se dissout pas.

3. Procédé selon la revendication 1, dans lequel l'étape 1) comporte l'utilisation d'une matrice microparticulaire, qui comprend l'agent polarisant approprié pour la PDN ou qui est constituée de celui-ci, et la mise en suspension, l'émulsification ou l'enduction de ladite matrice microparticulaire avec une solution, émulsion ou suspension comprenant les molécules présentant de l'intérêt, à une première température à laquelle la matrice microparticulaire ne se dissout pas et dans lequel l'étape 4) comporte l'augmentation de la température jusqu'à ce que la solution, émulsion ou suspension comprenant les molécules présentant de l'intérêt soit liquide et le retrait par filtration de la matrice microparticulaire, qui comprend l'agent polarisant approprié pour la PDN ou qui est constituée de celui-ci.

4. Procédé selon l'une quelconque des revendications précédentes, dans lequel les particules de la matrice microparticulaire ont une taille moyenne de particule dans la plage de 3-30 µm, de préférence dans la plage de 5-20 µm, de préférence en particulier dans la plage de 8-15 µm.

5. Procédé selon l'une quelconque des revendications précédentes, dans lequel la température dans les étapes 2) et/ou 3) est d'au maximum 10 K, de préférence d'au maximum 5 K, de préférence en particulier d'au maximum 2 K.

6. Procédé selon l'une quelconque des revendications précédentes, dans lequel le champ magnétique statique dans l'étape 3) est d'au moins 2 T, de préférence d'au moins 3 T ou 4 T, de préférence en particulier d'au moins 6 T.

7. Procédé selon l'une quelconque des revendications précédentes, dans lequel la polarisation croisée hétéronucléaire dans l'étape 3) comprend l'exposition pulsée et/ou continue à des radiofréquences essentiellement aux fréquences de Larmor à la fois des spins nucléaires abondants et du type de spin nucléaire différent, dans lequel de préférence le type de spin nucléaire différent est choisi dans le groupe constitué par ¹³C, ¹⁵N, ²⁹Si, ³¹P, ⁶Li et ⁸⁹Y et dans lequel encore de préférence les molécules présentant de l'intérêt sont enrichies en ce type de spin nucléaire différent.

8. Procédé selon l'une quelconque des revendications précédentes, dans lequel dans l'étape 4) les molécules présentant de l'intérêt sont séparées de l'agent polarisant, de préférence en ce que premièrement température est montée jusqu'à ce que la solution ou suspension formant du verre comprenant l'agent polarisant ou la molécule présentant de l'intérêt soit à l'état liquide mais que la matrice microparticulaire soit encore à l'état solide, pour permettre la séparation de la matrice microparticulaire de la solution ou suspension formant du verre ou au moins de l'agent polarisant contenu dans celle-ci, de préférence à l'aide d'une filtration, d'une centrifugation, d'une sédimentation, d'une chromatographie ou d'une association de celles-ci, et la dissolution de la matrice microparticulaire et/ou des molécules présentant de l'intérêt isolées.

9. Procédé selon l'une quelconque des revendications précédentes, dans lequel après l'étape 3) et avant l'étape 4) l'échantillon est transporté vers un endroit éloigné, ledit transport comportant le retrait temporaire de l'échantillon du champ magnétique de l'étape 3).

10. Procédé selon l'une quelconque des revendications précédentes, dans lequel la solution ou suspension comprenant un agent polarisant approprié pour la PDN comprend l'agent polarisant approprié pour la PDN à une concentration d'au moins 5 mM, de préférence d'au moins 10 mM, de préférence encore dans la plage de 10-50 mM, et/ou dans lequel l'agent polarisant approprié pour la PDN est choisi parmi les dérivés de nitroxydes, de préférence dans le groupe constitué par le TEMPO, le TEMPOL, le TEMPO-benzoate, mais également d'autres radicaux tels que le BDPA, le DPPH, le galvinoxyl ou un mélange de ceux-ci.

11. Procédé selon l'une quelconque des revendications précédentes, dans lequel un solvant et/ou liquide de mise en suspension est choisi dans le groupe constitué par le tétrahydrofurane, le dioxane, le butane, l'hexane, l'heptane, l'octane, le nonane, le benzène, le toluène, le DMSO, l'eau, le glycérol ou un mélange de ceux-ci.

12. Procédé selon l'une quelconque des revendications précédentes, dans lequel la molécule présentant de l'intérêt est choisie dans le groupe constitué par l'urée, l'ion pyruvate, l'ion fumarate, l'ion malate, le glucose, l'ion acétate, l'alanine ou un mélange de ceux-ci.

13. Procédé selon l'une quelconque des revendications précédentes, dans lequel dans l'étape 4) la molécule présentant de l'intérêt est dissoute dans de l'eau ou dans un liquide physiologique.
